# EUROPEAN PATENT SPECIFICATION

(11) **EP 4 217 054 B1**
(45) Date of publication and mention of the grant of the patent: **18.11.2026**
(21) Application number: 21777812.5
(22) Date of filing: 23.09.2021
(51) Int. Cl.: A61N 1/40, A61N 2/00, A61N 2/02

(54) **STIMULATION DEVICE FOR STIMULATING A NERVE**
STIMULATIONSVORRICHTUNG ZUR STIMULATION EINES NERVS
DISPOSITIF DE STIMULATION POUR STIMULER UN NERF

(30) Priority: 23.09.2020 CH 12082020; 19.10.2020 CH 13482020
(43) Date of publication of application: 02.08.2023
(73) Proprietor: Stimit AG, 2503 Biel (CH)
(72) Inventor: MÜLLER-BRUHN, Ronja, 5210 Windisch Aargau (CH); DEGEN, Thomas, 8903 Birmensdorf Zürich (CH); FENGELS, Dirk, 6037 Root (CH)
(74) Representative: Ruttensperger Lachnit Trossin Gomoll
(86) International application number: PCT/EP2021/076272
(87) International publication number: WO 2022/063933

(56) References cited:
- EP-A1- 1 442 766
- EP-A1- 3 355 987
- EP-B1- 3 355 987
- WO-A1-2015/179744
- WO-A1-2019/150378
- US-A1- 2011 207 988
- US-A1- 2012 016 280
- US-A1- 2014 024 882
- US-A1- 2017 326 357
- US-B2- 8 932 196

## Description

### Technical Field

The present invention relates to a stimulation device for stimulating a nerve in a human or animal body using an electro-magnetic field, thereby activating a target tissue in the human or animal body.

### Background Art

In medicine, it is known that for many purposes it is beneficial to activate a target tissue of a patient using stimulation by electro-magnetic fields. For achieving such activation of tissues in a patient's body, it is known to directly stimulate the tissue or to indirectly activate the tissue via stimulation of specific parts of the neural system. For example, the target tissue being a muscular tissue can be activated by providing electric pulses directly to the muscular tissue or to nerves associated to the muscular tissue.

In critical care units of hospitals, it may be desired to activate the diaphragm of ventilated patients in order to prevent drawbacks of disuse of the diaphragm. It was shown that disuse atrophy of diaphragm muscle fibres occurs already in the first 18-69 hours of mechanical ventilation, and the muscle fibre cross-sections decreased by more than 50% in this time. Thus, it is aimed to activate the diaphragm repeatedly while the patient is given artificial or mechanical respiration such that the functioning of the diaphragm can be upheld, or to activate the diaphragm at least during the weaning period to support effective restoration of independent respiratory function.

US 2016/0310730 A1 describes an apparatus for reducing ventilation induced diaphragm disuse in a patient receiving ventilation support from a mechanical ventilator. The apparatus includes an electrode array of first and second types and comprises a plurality of electrodes configured to stimulate a Phrenic nerve of the patient. At least one controller identifies a type of electrode array and generates a stimulus signal for stimulating a Phrenic nerve of the patient based upon the identity of the electrode type.

DE 10 2007 013531 A1 describes a magnet coil arrangement with at least two coil areas that are juxtaposed and largely mirror-symmetrical. The at least two coil areas are formed in a de-central manner for producing de-centralized fields in order that the electric field with highest strength being arranged close to the edge of the magnet coil arrangement.

EP 1 442 766 A1 describes pulsed electromagnetic field (PEMF) therapy that promotes healing of skeletal bones and other body tissues, and more particularly to a PEMF system having a high-efficiency single-coil transducer for providing PEMF therapeutic stimulation to a target area of a patient's body.

However, when two target nerves inside the body like the two Phrenic nerves shall be stimulated, the two coils shall not only be located in a close distance to each other but also operated simultaneously. The coil winding systems in today's stimulators used for electro-magnetic stimulation have significant limitations.

Moreover, the electro-magnetic fields generated by coil winding systems may have negative impact on the functionalities of the other medical devices located nearby. In the worst case, those medical devices can stop working properly, which should be definitely prevented.

Therefore, there is a need for a non-invasive stimulation device and respiration prompting apparatus avoiding or minimising the impact generated by electric o electro-magnetic stimulation for the other medical devices nearby. Further needs may include efficient stimulation of the two nerves, overcoming space constraints, avoiding co-stimulation effects of tissue in the vicinity of the nerves, simple application at a body as well as convenient and little painful application for the patient.

### Disclosure of the Invention

According to the invention this need is settled by a stimulation device as defined by the features of independent claim 1. Preferred embodiments are subject of the dependent claims.

In one aspect, the invention is a stimulation device to stimulate a first nerve and a second nerve in a human or animal body for activating a target tissue in the human or animal body. The stimulation device comprises a first coil unit, a second coil unit typically different or separable from the first coil unit and a security arrangement.

The first coil unit is configured to be positioned at the human or animal body to stimulate the first nerve by applying an electric or electro-magnetic first field. The second coil unit is configured to be positioned at the human or animal body to stimulate the second nerve by applying an electric or electro-magnetic second field. Thereby, the first coil unit can particularly generate the first field independent from the second coil unit generating the second field. By providing two independent fields, the first nerve and the second nerve can individually be stimulated by the first field and the second field, respectively. In other words, the first and second fields are independent and/or different from each other. Depending on the given situation in a specific application, the first and second field may still interact but they are independent and widely different from each other. For example, at least a portion of the first and second fields may overlap when the stimulation device is operated such that a region is generated in which both fields are present. Nevertheless, also in such situations the first and second fields are still independent and widely different from each other.

By having the first and second coil units configured to generate the first and second fields, an efficient coordinated stimulation of the first and second nerves can be achieved. For example, for promoting respiration, the first coil unit can be arranged at a neck of a patient to stimulate the first Phrenic nerve of the patient and the second coil unit to stimulate the second Phrenic nerve of the patient.

Further, the security arrangement is configured to ensure compliance with a security policy during stimulation of the first and second nerves by means of the first and second coil units. In this context, the term "configured" can relate to any suitable physical, logical or functional arrangement of the security arrangement. For example, the security arrangement, can have a processing unit which adapted or programmed to perform an evaluation of the given situation and to draw conclusions as to ensuring compliance. Additionally or alternatively, the security arrangement can comprise means such as sensors or the like to gather data related to the given situation.

In a variant, a stimulation device to stimulate a first nerve and a second nerve in a human or animal body for activating a target tissue in the human or animal body is suggested, which comprises a first coil unit configured to be positioned at the human or animal body to stimulate the first nerve by applying an electric or electro-magnetic first field, and a second coil unit configured to be positioned at the human or animal body to stimulate the second nerve by applying an electric or electro-magnetic second field; and which is configured to cancel a magnetic or electro-magnetic field in a region offset the first and second nerve when the first and second coil units are applying the first and second fields.

The term "position" and derivations thereof as used herein typically relates to a location and orientation of an element or component. If an element or component is positioned to be capable of doing something it advantageously is located and orientated to achieve the respective function. For example, the first and second coil units being positioned to stimulate the Phrenic nerves may relate to the first and second coil units being located and oriented such that the Phrenic nerves are within the electric or electro-magnetic field the respective coil unit generates.

The term "security policy" as used herein relates to at least one but advantageously plural constraints or conditions to be achieved. In particular, the constraints or conditions may be predefined. They can depend on the specific application of the device. For example, as described below, when the stimulation device is used in critical care units or in other medical rooms such as a surgery room, a constraint predefined in the security policy may be to minimize or eliminate disturbances of other equipment arranged in the same room by stimulation device.

The term "compliance" and derivations thereof as used in connection with the security policy can relate to meeting or fulfilling constraints or conditions predefined in the security policy.

The coil unit can be or comprise at least two coils or at least one cone shaped or otherwise curved or bulged coil, or at least one cylindrical or otherwise non-flat coil, or at least one small coil, i.e. a coil sufficiently small to generate a sharp electro-magnetic field such as a coil having a diameter of 3 cm or less. The targeted shape of the electro-magnetic field described herein can comprise a peak formed by the spatial electro-magnetic field. The electro-magnetic field generator can also be referred to as electro-magnetic field creator.

The first and second fields can particularly be targeted shape. The targeted shape can be achieved by the respective field being a locally constrained, targeted electric or electro-magnetic field, e.g., having a peak. It can be adapted to be active in a target area being the nerve area or tissue area that shall be activated with the respective field (e.g. the phrenic nerve that shall be activated), which can be for example achieved by the peak in the respective field (focality area). The targeted shape can generally be any shape of the respective field or the time-dependent field component that allows to stimulate one or more target nerves effectively while minimizing other undesired co-stimulation effects of surrounding, above-lying or close-by tissues or nerves. A peak shape is such example, because it maximizes effects in a focality area and minimizes effects outside this area.

In an advantageous embodiment, the stimulation device is configured to activate a diaphragm of a human or animal being. Thereby, the two nerves of the human and animal being may particularly be the Phrenic nerves and the coil units may be designed to be arranged at a neck of the human or animal being in order to stimulate the Phrenic nerves at the neck.

By having the security arrangement, the stimulation device of the invention can ensure that the security policy is complied with. In particular, it can be achieved that the security policy is met when operating the stimulation device. Like this, security during operating of the stimulation device may be increased or ensured. This allows the stimulation device being particularly suitable for medical applications such as activation of the diaphragm by stimulating the Phrenic nerves at a comparably sensible location such as the neck. The stimulation device according to the invention also allows for automatically taking an action if compliance cannot be assured. For example, if the security policy in not complied the stimulation device can interrupt stimulation or provide a signal such as a visual and/or acoustic signal.

Advantageously, the stimulation device is arranged to generate the first and second field in pulses or, more specifically, in single pulses, trains of pulses or combinations thereof. Thereby, the term "single pulses" can relate to the generation of the first or second field over a comparably short time and with a comparably long interruption between two subsequent pulses. Typically, single pulses are provided at frequencies lower than 10 Hertz (Hz) such as, e.g. at 5 Hz or below, or single pulses are initiated by the user or practitioner. The single pulses can have a temporal width of about 10 microseconds (µs) to about 300 µs. Such pulses can activate nerves and muscle structure and are identifiable by the patient or by a sensor. In particular, such single pulses may cause a single convulsion of a muscle or muscular structure. The term "trains of pulses* can relate to either a continuous generation of the first or second field or generation of a sequence of pulses of the first or second field comparably quickly following each other. Such pulses can be provided in a frequency range of in between about 15 Hz and about 30 Hz. In particular, a train may achieve to activate a nerve or muscle such that a tetanic contraction or activation is induced. Advantageously, the train is provided by increasing the intensity (field strength) and/or frequency until a target intensity and frequency is achieved (ramp protocol). Like this, sudden convulsion or discomfort can be decreased. All of these parameters are summarized under the term "temporal characteristics" or "temporal parameters" of the first and second fields. These temporal parameters can be adjusted manually via an input interface or be controlled automatically by an adjustment mechanism or control unit.

The parameters of the voltage or current waveform applied to generate the first and/or second field may affect the temporal characteristics of the respective field, including pulse shape, amplitude, width, polarity, and repetition frequency; duration of and interval between bursts or trains of pulses; total number of pulses; and interval between stimulation sessions and total number of sessions have, amongst others, an influence on the field strength and determine if and with which intensity or "dose" a target area or target tissue can be activated.

The temporal characteristics and spatial distribution of the first and/or second fields can be tuned in such a way that the desired activation (activation feedback) of the muscular structure is achieved. Thereby, the activation feedback (signal) may refer to a signal that indicates appropriate characteristics of muscular structure activation, e.g. a signal that reaches or exceeds a target value (threshold), a signal that exhibits a certain curve pattern or shape, a signal that fulfils a certain algorithm known to represent appropriate target muscular structure activation in the desired strength, or any combination thereof. The activation feedback (signal) may comprise a feedback in particular about a desired muscle activation strength that shall be reached before the adjustment mechanism stops variation. The appropriate activation feedback signal characteristics can for example be defined by a user via an input interface or be detected by algorithms.

The parameters of the voltage or current waveform applied to the coil by a generator affect the temporal characteristics of the first and/or second field, including pulse shape, amplitude, width, polarity, and repetition frequency; duration of and interval between bursts or trains of pulses; total number of pulses; and interval between stimulation sessions and total number of sessions have, amongst others, an influence on the field strength and determine if and with which intensity or "dose" a target area or target tissue can be activated.

Preferably, the security policy comprises cancelling an electric or electro-magnetic field in a region offset the first and second nerve when the first and second coil units are applying the first and second fields. The term "offset the first and second nerves" relates to any field or portion of a field not being required for stimulating the first and second nerves. It can particularly cover a far field generated by the first and second coil units.

Thereby, cancelling the field may cover essentially reducing or eliminating the field. More specifically, cancelling the field may involve to reducing the field in order that disturbances for other devices or apparatus may be excluded. Also, the complete field not required for stimulation can be cancelled or only portions thereof where disturbances may be caused.

By having such security policy implemented, the stimulation device can be suitable for being used or applied in vicinity of other devices or components, which may be prone for disturbances by electric or electro-magnetic fields. Like this, the stimulation device can particular be suitable for medical applications, e.g., in critical care units.

Preferably, the security arrangement comprises a support or bracket structure substantially axially arranging the first and second coil units, wherein the first coil unit comprises a first winding and the second coil unit comprises a second winding, and wherein in an axial view a stimulation current flows in opposite directions through the first and second winding, in the axial view of the first or second windings. In other words, the stimulation current direction is opposite to the second direction if regarded from a distinct point in space. In particular, the stimulation current flows clockwise in the first winding and counter-clockwise in the second winding or vice versa. The term "axial view" in this connection relates to a view or perspective along the axis along which the first and second coil units are arranged by means of the bracket structure. Such arrangement allows for efficiently cancelling the first and second fields such that side effects of generation of the first and second fields can be reduced or limited.

The different direction of current flows can be achieved by the first winding being wound in a first direction and the second winding being wound in a second direction and the first direction is opposite to the second direction. In other words, the first direction may be clockwise and the second direction may be counter-clockwise or vice versa.

The first and second coil units preferably are adjustable relative to the support structure such that an angle between an axis of the first coil unit and an axis of the second coil unit is 30° or less, or 20° or less, or 10° or less.

The axes of the coil units can particularly be axes about which the respective windings are wound. Thus, the axes can be central axes of the first and second coil units.

The electro and magnetic field strength decrease when the distance from the source increases. Usually, a far field relates to the electro and/or magnetic field with a distance to the coil that is approximately more than 10 times of dimension of the coil. In comparison a near field relates to the electro-magnetic field close to the coil. In the present invention, the far field describes the electro-magnetic field that may negatively interfere the other medical devices arrange around the stimulation device, whereas the near filed relates to the electro-magnetic field effectively used for stimulation of the nerves. Hence, the electro-magnetic field in the far field should be kept as small as possible in order to avoid the impact to the other medical devices during the stimulation. In the present invention, the near field can have a distance of 0.1 cm, 0.5 cm, or up to 4 cm to the coil, whereas the far field can have a distance of 30 cm or 45 cm to the coil.

By fixing or setting possible orientations of the first and second coil units relative to each other, it can be achieved that potentially disturbing fields are eliminated. More specifically, an electric or electro-magnetic field respectively generated by the first and second coil units can compensate each other offset the first and second fields. The sum electric or electro-magnetic field, i.e. the sum or aggregation of the first and second electric or electro-magnetic field can be reduced or minimised, thereby avoiding disturbing surrounding devices.

Preferably, the first and second coil units are arranged such that a sum of the first and second fields generated by the first and second coil units is about zero at the central axial point between the first and second coil units, or at a distance more than 30 or 45 cm from the first or second coil unit, i.e. in the so-called far field. This is particularly advantageous to reduce or avoid the impact of the electric or electro-magnetic fields for the other medical devices around the stimulation device. The sum of the first and second fields may still be effective in a distance of 0.1 to 4 cm, i.e. the near field. In other words, each of the first and fields is still effective for the stimulation. This is particularly advantageous to reduce or avoid the impact of the electro-magnetic fields for the other medical devices around the stimulation device.

Advantageously, a distance between the first and second coil units is defined in favour of the compensation or cancellation. The distance can vary when using the stimulation device for different patients. Preferably, the first and second coil units are placed nearby two nerves and the distance therebetween is depended on the body of the patient. The sum electric or electro-magnetic field may also depend on the strength of the stimulation current and the physical property of the coil unit. In an ideal case, the two fields could entirely compensate each other. In other words, the sum of the electric or electro-magnetic fields generated by the first and second coil units may be about zero at the point as described above.

Preferably, the security arrangement comprises a control unit. Thereby, the control unit can be or comprise any suitable computer or computing apparatus suitable for control of the stimulation device. In particular, the control unit can be embodied by a specific or a generic computing device. Or, it can be implemented in a computing device or structure included in another apparatus such as a ventilation apparatus or the like.

Computers or computing devices embodying the control unit may include a processor, a volatile and/or permanent memory, a data storage, a communication interface, a user interface, or the like. They typically are programmable or configurable.

The control unit can be configured to initiate the stimulation in accordance with the security policy which may include constraints or conditions to activate or deactivate the stimulation. If at least one of the conditions pr constraints defined in the security policy is not met, the stimulation may be deactivated or not activated at all. In other words, the control unit may prevent the stimulation being activated when the security requirements are not fulfilled. Accordingly, the first and second fields may not be generated by the coil units, thereby avoiding negative impact on the other medical devices arranged nearby the stimulation device.

The control unit of the security arrangement preferably is configured to detect a non-compliance with the security policy and to prevent activation of the first and second coil units when the non-compliance is detected. The non-compliance can be given when any constraint or condition of the security policy is not met.

For example, if the security policy defines that the first and second coil units have to be at a specific relative position, the security arrangement may be configured to verify the current relative position of the first and second coil units, wherein the control unit prevents activation of the first and second coil units if the current relative position does not comply with the specific relative position. The specific relative position may also comprise a range of relative positions in which the first and second coil units have to be arranged.

The control unit preferably is configured to activate the first and second coil units in case the security policy is met. Such embodiment allows for efficiently achieving operation of the stimulation device only if it complies with the security policy.

Preferably, the stimulation arrangement is configured to activate the first and second coil units by supplying a stimulation current to the first and second coil unit. Such activation of the first and second coil units allows for efficiently operating the stimulation device. In particular, such activation can precisely be performed by the control unit.

Preferably, the security arrangement comprises a sensor unit configured to determine positions of the first and second coil units relative to each other. The position information determined by the sensor unit can be used for calculation of the sum electro-magnetic field. In other words, the sum electro-magnetic field can be estimated using the position and orientation information without activating the stimulation. In this embodiment, the security policy may define one of the conditions to be met for the activation of the stimulation, i.e. the relative position and orientation needs to be within a certain range. The range can vary in the application of the stimulation device as well as the physical properties of the stimulation device. For instance, for a certain stimulation device the range for relative orientation can be ± 15° or 90° ± 15°. In any event, the range can be predefined or calculated for the certain stimulation device in a particular application. Evaluation of the determined positions can particularly be implemented in the control unit.

Thereby, the security policy preferably comprises the relative positions being within a predefined range. Like this, it can be assured that the coil units are only activated, when they are properly positioned. For example, the sensor unit allows for determining if they are more or less axially oriented in a predefined distance to each other. Like this, it can be ensured that the field is cancelled where not required.

The sensor unit may include any sensors appropriate to determine positions of the first and second coil units. For example, the sensor unit can comprise a gyroscope. Such gyroscope allows for measuring angular velocity in a comparably fast manner. Like this, whereas absolute angle measurements usually are not possible with gyroscopes, angular changes can be efficiently detected, e.g., by means of integration. Furthermore, gyroscopes are available at comparably low costs and in comparably small dimensions.

Alternatively or additionally, the sensor unit comprises an accelerometer. Such an accelerometer or three axis accelerometer allows for comparably precise angular measurement and particularly absolute angular measurement such that conclusions about the positions of the coil units can be drawn. Furthermore, accelerometers are available at comparably low costs and in comparably small dimensions.

Alternatively or additionally, the sensor unit comprises a magnetometer. Such magnetometer allows for comparably precisely measuring a three-dimensional orientation of the sensor such that conclusions about the orientation of the intervention instrument can be drawn.

Alternatively or additionally, the sensor unit comprises another inertial sensor and/or a global positioning system.

The sensor unit may also have two parts each to be associated to one of the coil units. Furthermore, the sensor unit may comprise a camera, such as a stereo-camera to determine relative position by observance.

The security arrangement comprises a measurement unit configured to measure a first magnetic flux of the first coil unit and a second magnetic flux of the second coil unit. Measuring such flux allows for implementing specific security measures. For example, the flux may be indicative for the status of the coil units. Thereby, evaluating the flux allows for identifying a damaged or inappropriate coil unit. Thus, the measurement unit allows for increasing operation safety of the stimulation device.

Thereby, the security policy preferably comprises preventing a sum of the first and second magnetic fluxes exceeding a predefined threshold, e.g. a few milli tesla. For example, the control unit can be configured to stop or prevent activation of the coil units when the sum of the first and the magnetic flux density exceeds the predefined threshold. Like this, a further increased safety can be achieved.

The stimulation device preferably is configured to measure the first and second magnetic fluxes by measuring a current flowing through the first and second coil units. Thereby, an electric current flowing through the first and second coil units can be used for the measurement. This can be, for instance, a specific measurement current that is sufficiently small so that the electro-magnetic fields generated thereby do not interfere other medical devices or harm the patient but it still sufficient for the measurement and calculation of the sum electro-magnetic field.

The stimulation device preferably is configured to measure the first and second magnetic fluxes by using a first part of a plurality of consecutive waveforms of a stimulation current, wherein the security arrangement is configured to deactivate stimulation when the sum of the first and the magnetic flux exceeds the predefined threshold. Thereby, the electric current for the measurement can be the initial part of the stimulation current for a short time period but still sufficient for the measurement. The measurement can be performed during the initiation process of the stimulation device which should be short, e.g. few milliseconds to few hundreds of milliseconds. Once the result of the measurement shows that the sum magnetic flux density exceeds the predefined threshold, the stimulation can be stopped at the initiation stage, i.e. the stimulation will not be activated.

Preferably, the security arrangement comprises a monitoring unit configured to detect a fault of the first coil unit and/or the second coil unit. The fault may be a defect coil winding, or a short circuit between turns or to ground. Additionally, a wrong value of the inductance could be a fault. For instance, a very low inductance may lead to a high current and thereby damaging the stimulation device and patient. Depending on the application the inductance should have a threshold which it may not below, e.g. the minimum inductance should not below 10% of the specified or predefined valve.

Thereby, the security policy preferably comprises the monitoring unit not detecting the fault of the first coil unit and/or the second coil unit. In this embodiment, the condition defined in the security policy may be that the first and second coil units are in working order.

Preferably, the stimulation device comprises a connector configured to electrically connect with the first and second coil units in series. The electric serial connection of the first and second coil units may facilitate a simple activation and control of a simultaneous stimulation at the two nerves. Conventionally, the two coils are controlled separately, thereby the simultaneous stimulations at the two nerves require simultaneous activation of the two coils, which can be in same use case challenging. In comparison, by connecting the first and second coil units in series, the stimulation at the two nerves will be activated at the same time, thereby no additional measure for controlling a simultaneous activation of the stimulation is needed.

A time-synchronous stimulation of both nerves has advantages to allow time-synchronous activation of both nerves thus balanced contraction of both diaphragm hemispheres. The time-synchronous stimulation of both nerves has further advantages to minimize an electro-magnetic far field which is only possible when current in both coils is induced in exact timely synchrony and when the induced time-variant magnetic fields which are at its high exactly at the same time oppose each other in direction, or almost oppose each other within +/- 20°.

Preferably, the connector comprises an electrical conduct directly connecting the first and second coil units. The electrical conduct can be embodied by any electrically conducting structure such as a metal wire or the like. Such electrical conduct allows for efficiently connecting the first and second coil units in series.

Preferably, the first and second coil units are individually positionable at the human or animal body. The term "individually positionable" is directed to placing the coil individually at the body. Thus, the coil units are not commonly positioned at one single location at the body but at two locations separate from each other. However, the coil units can be individually positionable but still being fixed to each other. Or, the coil units can, at least to a certain extent, be movable relative to each other.

The stimulation device can comprise a holder structure for holding the coil units. In particular, the holder structure may have a bracket, a brace or a similar element to which the coil units are fixedly or displaceably mounted. By the holder, the first and second coil units can be mechanically connected with each other only. Another mechanical coupling directly between the first and second coil units, that may prevent or limit the relative arrangement of them, can be prevented. Thereby, the first and second coil units can be individually positioned at the human or animal body.

Preferably, the stimulation is configured such that each of the first and second coil units generates biphasic impulses.

Further, the stimulation device preferably is configured to generate time-variant currents in the first and second coil units being in timely synchrony.

Preferably, the stimulation device comprises a stimulator unit configured to induce currents in the coil units.

Preferably, the stimulation device is configured such that an induced current in the first coil is the same as an induced current in the second coil. For example, this can be implemented by arranging the first and second coil units electrically in series.

Preferably, the stimulation device is configured to induce impulses generated by one current.

Preferably, the security arrangement comprises a first temperature sensing formation and a second temperature sensing formation, and the first coil unit is equipped with the first temperature sensing formation and the second coil unit is equipped with the second temperature sensing formation. Such temperature sensing formations allow for determining the temperatures of the first and second coil units. This can be security relevant when operating the device.

The term "temperature sensing formation" relates to any structure and/or configuration allowing to sense a temperature. It can be a multipart construction or a single element.

Thereby, preferably the first temperature sensing formation comprises a first temperature sensor and a second temperature sensor, and the second temperature sensing formation comprises a first temperature sensor and a second temperature sensor. All the temperature sensors advantageously are operatable with the same voltage. Further, they advantageously have two outputs for forwarding sensed temperatures to other components. Providing each coil unit with two temperature sensors allows for achieving a high level security mechanism.

The security policy preferably comprises current consumptions of the first temperature sensor of the first temperature sensing formation, of the second temperature sensor of first temperature sensing formation, of the first temperature sensor of the second temperature sensing formation, and of the second temperature sensor of the second temperature sensing formation being within a predefined threshold range of current consumption. Such configuration allows for an efficient security check of the operation of the first and second coil units. Such predefined threshold range of current consumption can be, e.g., within 80% and 120% of the nominal value.

The security policy preferably comprises temperatures measured by the first temperature sensing formation and by the second temperature sensing formation being below a predefined threshold temperature such as e.g., 41°C. Such configuration allows for preventing overheating of the coil units which may be indicative for an inappropriate operation of the device.

Preferably, the security arrangement comprises a counter member configured to count a number of pulses induced by the first coil unit and second coil unit. The counter members can comprise a splitter or a plug. It can store the or determine the number of pulses of the first and second fields provided to a patient. By means of the counter member it can be assured that the patient is not constantly induced to inhale.

Thereby, the security policy preferably comprises the counted numbers of pulses per time being below a predefined threshold pulse number.

Preferably, a button is provided configured to, when activated, operate the first coil unit to induce a pulse of the first field when activated and to operate the second coil unit to induce a pulse of the second field. The button advantageously is provided with two members or touch feelers which both have to be pressed or touched to activate the button.

The above discussed conditions for activation the stimulation device can be extended and/or modified in case of need. For instance, if there are no medical devices nearby that are electro-magnetic field sensitive, the predefined threshold for the sum magnetic flux density can be increased, or this condition can be deleted or disabled from the security policy. When the stimulation device is used in another environment with electro-magnetic field sensitive medical devices, this condition can be added or enabled again. Also, other conditions be added in case of need, e.g. a maximum, minimum, or period of the stimulation intensity. For instance, if the stimulation device is supposed to be used for a patient who physical constitution should not be stressed with stimulation exceeding certain strength. In this case the stimulation device will check the current configuration and compares that with the maximum strength, prior to activating the stimulation.

In another aspect, however not forming part of the claimed invention, the description provides an exemplary method of stimulating first and second nerves in a human or animal body for activating a target tissue in the human or animal body. The method comprises the steps of: positioning a first coil unit at the first nerve of the human or animal body to stimulate the first nerve by applying an electric or electro-magnetic first field; positioning a second coil unit at the second nerve of the human or animal body by applying an electric or electro-magnetic second field; and ensuring compliance with a security policy during stimulation of the first and second nerves by means of the first and second coil units.

The exemplary method described above and its preferred embodiments described below allow for achieving the effects and benefits described above in connection with the stimulation device according to the invention and its preferred embodiments.

Preferably, the method comprises a step of adjusting the position of the first and second coil unit.

Preferably, the method comprises step of adjusting a stimulation current supplied to the first and second coil units.

Preferably, the security policy comprises cancelling an electric or electro-magnetic field in a region offset the first and second nerve when the first and second coil units are applying the first and second fields.

Preferably, the method comprises a step of substantially axially arranging the first and second coil units, wherein the first coil unit comprises a first winding wound in a first direction, the second coil unit comprises a second winding wound in a second direction and the first direction is opposite to the second direction.

Thereby, an angle between an axis of the first coil unit and an axis of the second coil unit preferably is 10° or less, or 5° or less.

Preferably, the first and second coil units are arranged such that a sum of the first and second fields generated by the first and second coil units is about zero.

Preferably, the method comprises a step of detecting a non-compliance with the security policy and preventing activation of the first and second coil units when the non-compliance is detected.

Preferably, the method comprises a step of activating the first and second coil units in case the security policy is met.

Preferably, the method comprises a step of activating the first and second coil by supplying a stimulation current to the first and second coil units.

Preferably, the method comprises a step of determining positions of the first and second coil units relative to each other.

Thereby, the security policy preferably comprises the relative positions being within a predefined range.

Preferably, the method comprises a step of measuring a first magnetic flux of the first coil unit and a second magnetic flux of the second coil unit.

Thereby, the security policy preferably comprises preventing a sum of the first and the magnetic flux exceeding a predefined threshold.

The first and second magnetic fluxes preferably are measured by measuring a current flowing through the first and second coil units.

The method preferably comprises a step of measuring the first and second magnetic fluxes by using a first part of a plurality of consecutive waveforms of a stimulation current, and deactivating stimulation when the sum of the first and the magnetic flux exceeds the predefined threshold.

Preferably, the method comprises a step of detecting a fault of the first coil unit and/or the second coil unit.

Thereby, the security policy preferably comprises not detecting the fault of the first coil unit and/or the second coil unit.

Preferably, the first and second coil units are connected in series.

Preferably, the method comprises a step of individually positioning the first and second coil units at the human or animal body.

Preferably, the method comprises a step of sensing a temperature at the first coil unit and a temperature at the second coil unit.

Thereby, the security policy preferably comprises sensed temperatures being below a predefined threshold temperature.

Preferably, the method comprises a step of providing first coil unit with a first temperature sensor and a second temperature sensor, and providing the second coil unit with a first temperature sensor and a second temperature sensor.

Thereby, the security policy preferably comprises current consumptions of the first temperature sensor of the first temperature sensing formation, of the second temperature sensor of first temperature sensing formation, of the first temperature sensor of the second temperature sensing formation, and of the second temperature sensor of the second temperature sensing formation being within a predefined threshold range of current consumption.

Preferably, the method comprises a step of counting a number of pulses induced by the first coil unit per time and counting a number of pulses induced by the second coil unit per time.

Thereby, the security policy preferably comprises the counted numbers of pulses per time being below a predefined threshold pulse number.

Preferably, the method comprises a step of manually operating the first coil unit to induce a pulse of the first field and manually operating the second coil unit to induce a pulse of the second field.

Preferably, in the method, a stimulation device as described above is used.

In a further other aspect, however not forming part of the claimed invention, there is provided a respiration promoting apparatus or ventilation machine to coordinately stimulate two Phrenic nerves of a patient for activating a diaphragm of the patient. The respiration promoting apparatus comprises a stimulation device as described above.

### Brief Description of the Drawings

The stimulation device according to the invention is described in more detail herein below by way of exemplary embodiments and with reference to the attached drawings, in which:
- Fig. 1: shows a schematic view of a stimulation device according to the invention;
- Fig. 2a: shows a schematic view of the electric connectivity of a stimulation device according to the invention;
- Fig. 2b: shows a schematic view of a stimulation device according to the invention arranged at the neck of a patient;
- Fig. 2c: shows another schematic view of the electric connectivity of a stimulation device according to the invention;
- Fig. 3a: shows a schematic top view of the first and second coil units being positioned at a neck of a patient, where the coil windings are arranged with their cylindrical circumference on the body surface;
- Fig. 3b: shows a schematic top view of the first and second coil units being positioned at the neck of the patient, where the coil windings are arranged with their outermost winding on the body surface;
- Fig. 4a: shows a schematic view of the arrangement of the first and second coil windings at the neck of a patient;
- Fig. 4b: shows a schematic view of the arrangement of the first and second coil windings at the nerves of a patient;
- Fig. 5: shows a schematic view of the arrangement of the first and second coil units with a preferred direction of the electro-magnetic fields in favour of cancelling themselves;
- Fig. 6: shows a schematic view of a respiration prompting apparatus comprising a stimulation device according to the invention; and
- Fig. 7: shows a schematic flow diagram illustrating the operation of a stimulation device.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

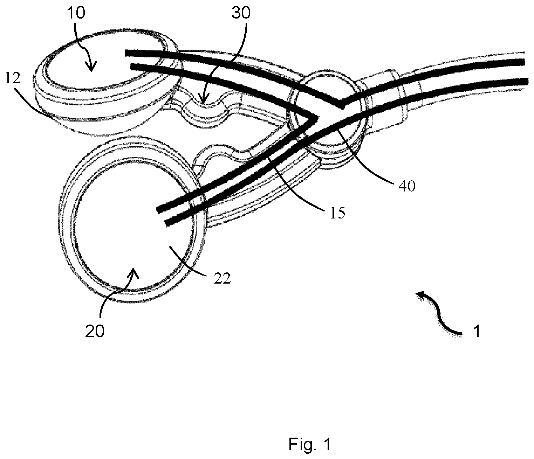

Fig. 1 illustrates an exemplary embodiment of a stimulation device 1 for coordinately stimulating the Phrenic nerves at a neck of a human patient, thereby activating a diaphragm of the patient as target tissue. The stimulation device 1 comprises a first coil unit 10, a second coil unit 20 and a connector with a metal wire 15 as electric conduct. The wire 15 directly couples the first and second coil units 10, 20 such that they are electrically connected in series.

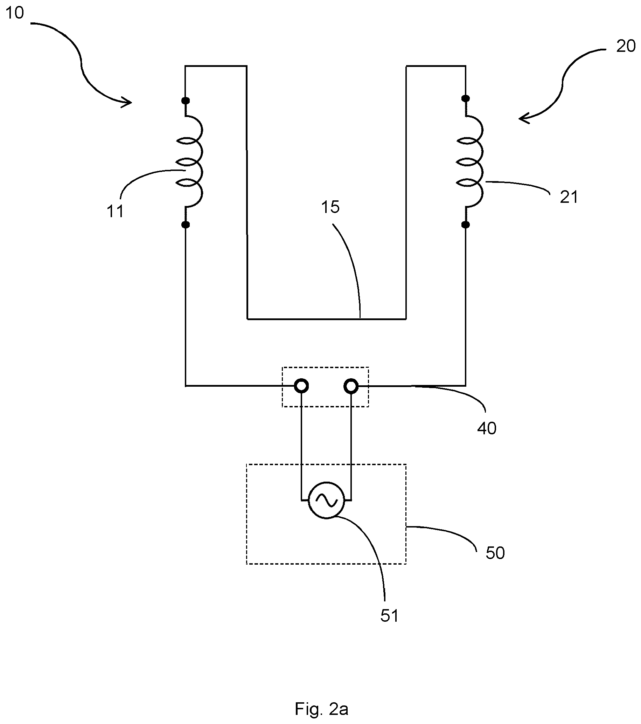

As shown in Fig. 2a, the first coil unit 10 comprises a first coil winding 11 for generating a first electro-magnetic field to stimulate a first one of the Phrenic nerves. The second coil unit 20 comprises a second coil winding 21 for generating a second electro-magnetic field to stimulate a second one of the Phrenic nerves. The first and second coil units 10, 20, each has a housing in which the first and second coil windings 11, 21 are arranged, i.e. enclosed, respectively. In order to increase the strengthen of the electro-magnetic field, each of the first and second coil units 10, 20 can also be provided with a plurality of coil windings 11, 21.

As depicted in Fig. 1, in order that the stimulation device 1 can be used at difference sized or formed bodies, the first 10 and second coil unit 20 can be individually positioned at the body or neck. In particular, for that purpose, the stimulation device 1 has a bracket structure 30 as support structure or holder which mechanically couples the first 10 and second coil unit 20. As shown in Fig. 1, the first 10 and the second coil unit 20 are mechanically connected with each other only via the bracket structure 30. More specifically, the bracket structure 30 comprises two arms connected at one of their longitudinal ends via a joint member. An angle between the arms is adjustable at the joint member. At longitudinal end opposite to the joint member, the first and second coil units 10, 20 are pivotably mounted to the arms of the bracket structure 30. This facilitates the individual positioning of the coil units 10, 20 at the body of the human and, in particular, at a neck of the human.

Fig. 2a schematically shows an electric circuit of the stimulation device 1, where the first and second coil windings 11, 21 are connected in series via the wire 15 of the connector. The serial connection further comprises a power supply 51 of a control unit 50 that is connected with the coil windings 11, 21 via further wires 40 of the connector in series. As mentioned, the first and second coil windings 11, 21 are directly connected via the wire 15 which is also a part of the serial connection. The wire 15 is arranged within the bracket 30.

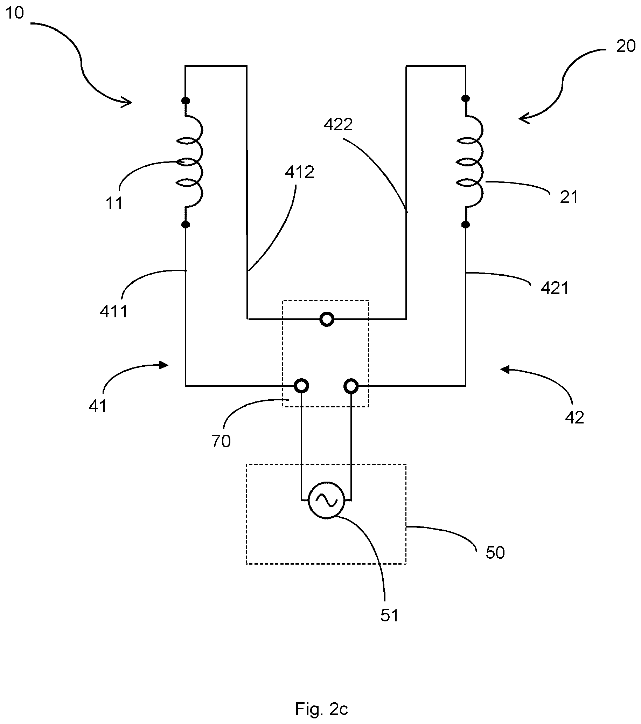

Fig. 2c schematically shows an alternative electric circuit of the stimulation device 1, where the first and second coil windings 11, 21 are connected in series via a splitter 70. In particular, the first coil windings 11 are connected to the splitter by means of first electrical cable 41 which has a forward electrical conduct 411 and a backward electrical conduct 412. Similarly, the second coil windings 21 are connected to the splitter 70 by means of second electrical cable 42 which has a forward electrical conduct 421 and a backward electrical conduct 422.

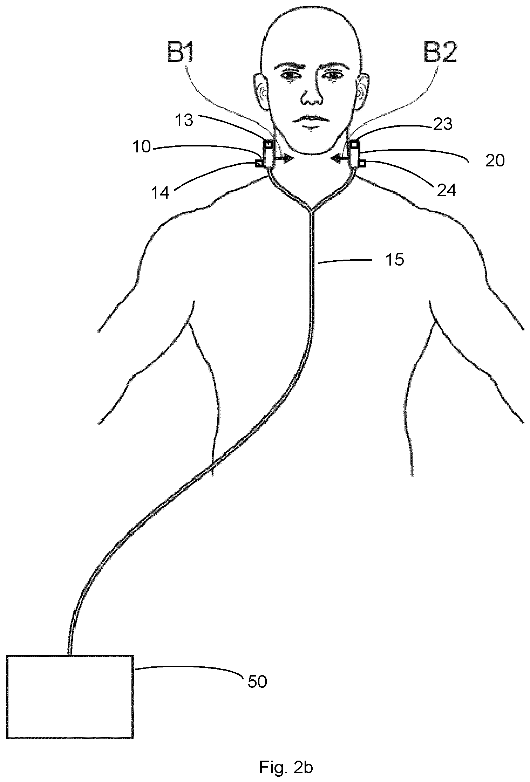

Fig. 2b schematically shows the stimulation device 1 arranged at the Phrenic nerves. In particular, the first coil unit 10 generates a first electro-magnetic field B1 for stimulate a first Phrenic nerve and the second coil unit 20 generates a second electro-magnetic field B2 for stimulate a second Phrenic nerve. As depicted the first and the second coil units 10, 20 are connected in series via the wire 15. The first and second coil units are arranged, in view of the axes of the first 11 and second coil windings 21, substantially axially to each other. Further, the stimulation current flows through the coil windings in opposite directions (not shown). Hence, the electro-magnetic field B1 generated by the first coil unit 10 have the opposite direction as that of the electro-magnetic field B2 generated by the second coil unit. In an ideal case the electro-magnetic fields B1 and B2 can cancel themselves at the places that are 30 cm or 45 cm or more distanced, thereby avoiding interference to the other medical device. At the same time, the electro-magnetic fields B1 and B2 are still effective for the stimulation, i.e. at the nerves of the patient, which are close to the coil units 10, 20, e.g. 0.1 cm, 0.5 cm, or up to 4 cm.

Furthermore, the first coil unit 10 is equipped with a first double temperature sensor 13 of a first temperature sensing formation and a first push member 14 of a button. The second coil unit 20 is equipped with a second double temperature sensor 23 of a second temperature sensing formation and a second push member 24 of the button. The security policy of the stimulation device comprises current consumptions of the first temperature sensor 13, of the second temperature sensor 23. Moreover, the security policy comprises temperatures measured by the first temperature sensor 13 and by the second temperature sensor 23 being below a predefined threshold temperature. Simultaneously, pressing the first and second push members 14, 24 activates the button such that the first and second coil units 10, 20 provide a pulse.

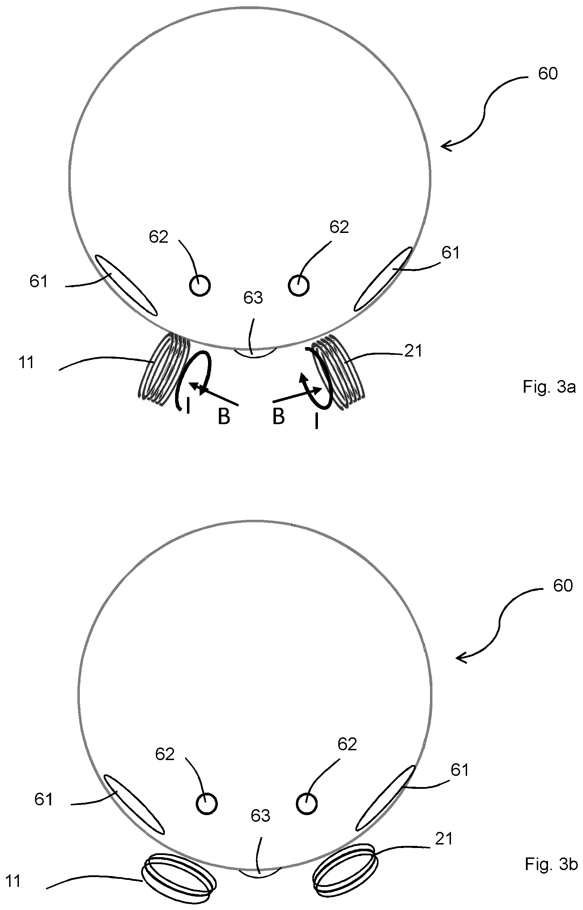

Fig. 3a shows an exemplary arrangement of the coil units, where the first and second coil windings 11, 21 are positioned on the skin of both sides of the patient's neck near the Phrenic nerves 61, 62 of the patient. In other words, each of the coil windings 11, 21 is placed between an anterior border of a right sternocleidomastoid muscle 61 and a larynx 63. The optimal position may vary according to varying physiologies of different patients and different treatments provided for the patient. In this example, the first and second coil windings 11, 21 are arranged with their cylindrical circumference on the neck surface, i.e. the axes of the coil windings are parallel to the neck. In other word, the coil windings 11, 21 are substantially aligned axial. When the coil windings 11, 21 are also winded axially in opposite directions, the electro-magnetic fields generated by the first and second coil windings 11, 21 can compensate each other, i.e. the sum of the first and second electro-magnetic fields decrease, i.e. the value of the aggregated electro-magnetic field is smaller than the first or the second electro-magnetic field. In an ideal case, that is when the first and second coil windings 11, 21 are axially symmetric and have a certain distance, first and second electro-magnetic fields compensate each other entirely, i.e. the sum of the first and second electro-magnetic fields is zero. This is for example the anti-Helmholtz coil.

Fig. 3b shows another exemplary arrangement of the coil units 10, 20 of the stimulation device 1, where the first and second coil windings 11, 21 are positioned at the skin of the both sides of the patient's neck that is facing to the nerves 61, 62. In contrast to the embodiment illustrated in Fig. 3a, the coil windings 11, 21 are arranged with their outermost winding on the surface of the neck. In this example, the axes of the coil windings 11, 21 are more or less perpendicular to the skin of the body and substantially parallel to each other.

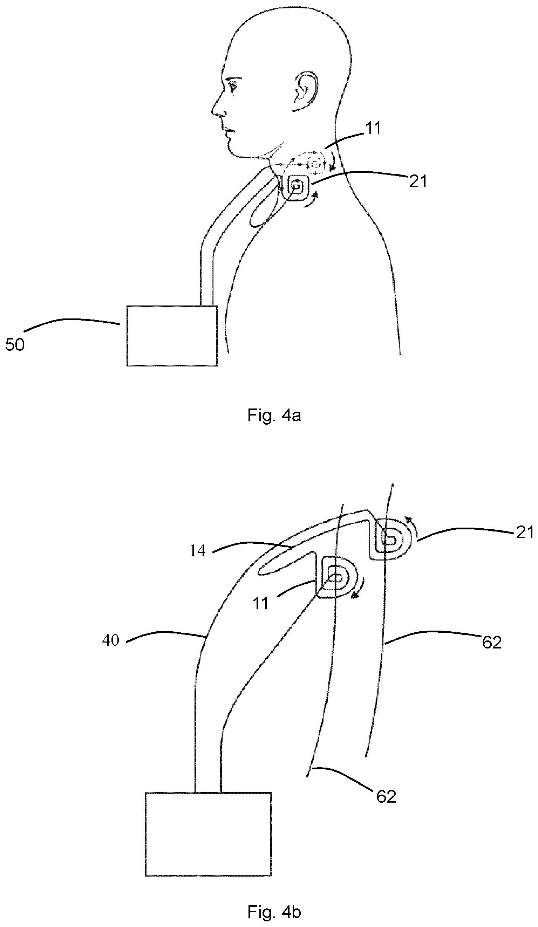

Fig. 4a shows an exemplary arrangement of the first coil winding 11 and the second coil winding 12 at the neck 62 of the patient. For better understanding the first 10 and the second coil units 20 are not completely shown. In this embodiment, the stimulation current flows in opposition directions in the first and the second coil windings. This can be for instance achieved by winding the first and second coil windings in opposite directions, as shown by the two arrows in the drawing. The coil windings 11, 21 are connected to the control unit 50. The electric current can be supplied by the control unit 50 to the coil windings for generating the electro-magnetic fields, thereby stimulating the nerves in the neck of the patient.

Fig. 4b shows an exemplary arrangement of the first coil winding 11 and the second coil winding 12 at the nerves 62 of the patient. For better understanding the first 10 and the second coil units 20 are not shown. In this embodiment, the stimulation current flows in opposition directions in the first and the second coil windings. This can be for instance achieved by winding the first and second coil windings in opposite directions, as shown by the two arrows.

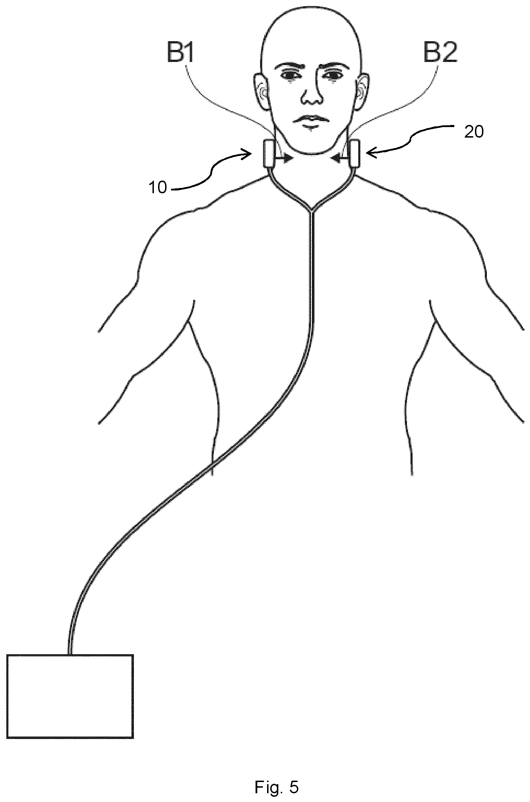

Fig. 5 shows an exemplary arrangement of the coil units 10, 20 of the stimulation device 1, where the first and second coil units are arranged, in view of the axes of the first 11 and second coil windings 21, substantially axially to each other. Further, the stimulation current flows through the coil windings in opposite directions (not shown). Hence, the electro-magnetic field B1 generated by the first coil unit 10 have the opposite direction as that of the electro-magnetic field B2 generated by the second coil unit. In an ideal case the electro-magnetic fields B1 and B2 can cancel themselves at the places that are 30 cm or 45 cm or more distanced, thereby avoiding interference to the other medical device. At the same time, the electro-magnetic fields B1 and B2 are still effective for the stimulation, i.e. at the nerves of the patient, which are close to the coil units 10, 20, e.g. 0.1 cm, 0.5 cm, or up to 4 cm.

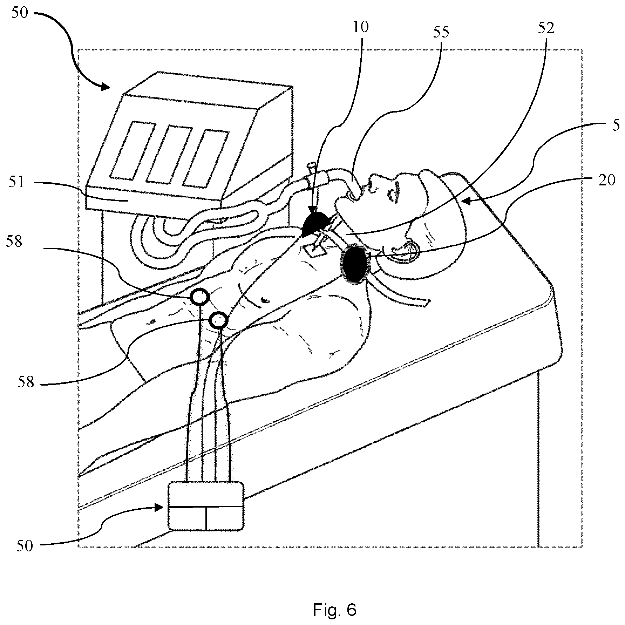

Fig. 6 shows a respiration promoting apparatus including a stimulation device according to the invention. First and second coil units 10, 20 are placed near the two Phrenic nerves of a patient. When the stimulation starts, a control unit 50 provides an electric current to the coil units thereby generating electro-magnetic fields that stimulate the Phrenic nerves. Consequently, the diaphragm of the patient is activated. The movement of the diaphragm 58 creates an under-pressure in the patient's chest cavity, which prompts the airflow from the respiratory tube 55. The control unit 50 monitors the movement of the diaphragm 58 and can adjust the intensity of the stimulation if needed.

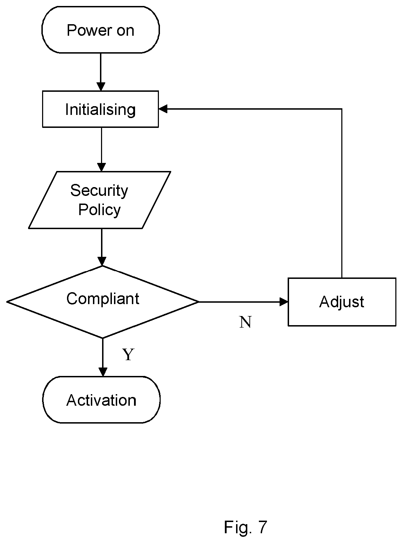

Fig. 7 shows an example of operating the stimulation device 1 described above. Upon powering on the stimulation device 1 will be initialised. During the initialising process, the current device parameters and status need to be checked with the security policy.

For instance, in order to verify the arrangement of the first and second coil units 10, 20 with the conditions in the security policy, the relative position and orientation of the first and second coil units 10, 20 are measured. If the results are within the range defined in the security policy the stimulation can be started.

Alternatively or additionally, the sum magnetic flux of the first and second coil units 10, 20 is measured. If the value does not exceed a predefined threshold, the stimulation can be activated. This security measure prevents the stimulation impacting the other medical devices arranged nearby.

Similarly, the coil windings 11, 21 can be also verified before the activation, which can be done by the measurement of some of the following parameters of the coil winding: inductance, resistance, impedance, leakage current to the grounding conductor. The impedance is the voltage divided by current. The inductance is the imaginary part of the resistance, the resistance is the real part of the impedance. Also starts of other electric components may be verified prior to activation of the stimulation. This security measure for the stimulation device can be seen as an automatic self-checking.

Once the control unit 50 confirms that the current setup of the stimulation device is compliant with the security policy, i.e. all conditions defined in the policy have been met, the stimulation can be activated. If one or more certain conditions are not met, the stimulation will not be started. Instead of that the operator may be prompted for adjusting the arrangement of the coil units 10, 20, the stimulation current or any other configuration. After the adjustment, the stimulation device may be re-initialised, i.e. the adjusted setup will be verified again with the security policy.

In addition, each of the first and second coil units 10, 20 can generate biphasic current. Further, the time-variant currents of the first and second coil units are in timely synchronised. The currents may be induced by one stimulator unit, wherein the induced current in the first coil is the same as the induced current in the second coil, and wherein the induced impulses are generated by one current.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

A computer program for operating the control unit 50 may be stored/distributed on a suitable medium, such as an optical storage medium or a solid-state medium supplied together with or as part of other hardware, but may also be distributed in other forms, such as via the Internet or other wired or wireless telecommunication systems. In particular, e.g., a computer program can be a computer program product stored on a computer readable medium which computer program product can have computer executable program code adapted to be executed to implement a specific method such as the method described above. Furthermore, a computer program can also be a data structure product or a signal for embodying a specific method such as the method described above.

## Claims

1. A stimulation device (1) to stimulate a first nerve (62) and a second nerve (62) in a human or animal body for activating a target tissue in the human or animal body, comprising:
a first coil unit (10) configured to be positioned at the human or animal body to stimulate the first nerve (62) by applying an electric or electro-magnetic first field;
a second coil unit (20) configured to be positioned at the human or animal body to stimulate the second nerve (62) by applying an electric or electro-magnetic second field; and
a security arrangement configured to ensure compliance with a security policy during stimulation of the first nerve (62) by means of the first coil unit (10) and of the second nerve (62) by means of the second coil unit (20),
**characterized in that** the security arrangement comprises a measurement unit configured to measure a first magnetic flux of the first coil unit (10) and a second magnetic flux of the second coil unit (20).

2. The stimulation device (1) of claim 1, wherein the security policy comprises cancelling an electric or electro-magnetic field in a region offset the first and second nerve (62) when the first coil unit (10) and the second coil unit (20) are applying the first and second fields.

3. The stimulation device (1) of any one of the preceding claims, wherein the first coil unit (10) and the second coil unit (20) are arranged such that a sum of the first and second fields generated by the first coil unit (10) and the second coil unit (20) is about zero at a distance more than 30 or 45 cm.

4. The stimulation device (1) of any one of the preceding claims, wherein the security arrangement comprises a control unit.

5. The stimulation device (1) of claim 4, wherein the control unit of the security arrangement is configured to detect a non-compliance with the security policy and to prevent activation of the first coil unit (10) and the second coil unit (20) when the non-compliance is detected, and/or to activate the first coil unit (10) and the second coil unit (20) in case the security policy is met.

6. The stimulation device (1) of any one of the preceding claims, configured to activate the first coil unit (10) and the second coil unit (20) by supplying a stimulation current to the first coil unit (10) and the second coil unit (20).

7. The stimulation device (1) of any one of the preceding claims, wherein the security arrangement comprises a sensor unit configured to determine positions of the first coil unit (10) and the second coil unit (20) relative to each other, wherein the security policy preferably comprises the relative positions being within a predefined range.

8. The stimulation device (1) of any one of the preceding claims,
wherein the security policy comprises preventing a sum of the first and the second magnetic flux exceeding a predefined threshold, and/or
wherein the stimulation device (1) is configured to measure the first and second magnetic fluxes by measuring a current flowing through the first coil unit (10) and the second coil unit (20), and/or
wherein the stimulation device (1) is configured to measure the first and second magnetic fluxes by using a first part of a plurality of consecutive waveforms of a stimulation current, wherein the security arrangement is configured to deactivate stimulation when the sum of the first and the magnetic flux exceeds the predefined threshold.

9. The stimulation device (1) of any one of the preceding claims, wherein the security arrangement comprises a monitoring unit configured to detect a fault of the first coil unit (10) and/or the second coil unit (20), wherein the security policy preferably comprises the monitoring unit not detecting the fault of the first coil unit (10) and/or the second coil unit (20).

10. The stimulation device (1) of any one of the preceding claims, further comprising
a connector configured to electrically connect with the first coil unit (10) and the second coil unit (20) in series, and/or
a button configured to, when activated, operate the first coil unit to induce a pulse of the first field and to operate the second coil unit to induce a pulse of the second field.

11. The stimulation device (1) of any one of the preceding claims, wherein the first coil unit (10) and the second coil unit (20) are individually positionable at the human or animal body.

12. The stimulation device (1) of any one of the preceding claims, configured such that each of the first coil unit (10) and the second coil unit (20) generates biphasic impulses.

13. The stimulation device (1) of any one of the preceding claims,
configured to generate time-variant currents in the first coil unit (10) and the second coil unit (20) being in timely synchrony, and/or
comprising a stimulator unit configured to induce currents in the first coil unit (10) and the second coil unit (20), and/or
configured such that an induced current in the first coil unit (10) is the same as an induced current in the second coil unit (20), and/or
configured to induce impulses generated by one current.

14. The stimulation device (1) of any one of the preceding claims, wherein the security arrangement comprises a first temperature sensing formation and a second temperature sensing formation, and wherein the first coil unit (10) comprises the first temperature sensing formation and the second coil unit (20) comprises the second temperature sensing formation,
wherein the first temperature sensing formation preferably comprises a first temperature sensor and a second temperature sensor, and wherein the second temperature sensing formation comprises a first temperature sensor and a second temperature sensor, in particular wherein the security policy preferably comprises current consumptions of the first temperature sensor of the first temperature sensing formation, of the second temperature sensor of first temperature sensing formation, of the first temperature sensor of the second temperature sensing formation, and of the second temperature sensor of the second temperature sensing formation being within a predefined threshold range of current consumption;
and/or
wherein the security policy preferably comprises temperatures measured by the first temperature sensing formation and by the second temperature sensing formation being below a predefined threshold temperature.

15. The stimulation device (1) of any one of the preceding claims, wherein the security arrangement comprises a counter member configured to count a number of pulses induced by the first coil unit (10) and the second coil unit (20), wherein the security policy preferably comprises the counted numbers of pulses per time being below a predefined threshold pulse number.

## Patentansprüche

1. Eine Stimulationsvorrichtung (1) zur Stimulation eines ersten Nervs (62) und eines zweiten Nervs (62) in einem menschlichen oder tierischen Körper zum Aktivieren eines Zielgewebes in dem menschlichen oder tierischen Körper, umfassend:
eine erste Spuleneinheit (10), die dazu ausgebildet ist, an dem menschlichen oder tierischen Körper positioniert zu werden, um den ersten Nerv (62) durch Anlegen eines ersten elektrischen oder elektromagnetischen Feldes zu stimulieren;
eine zweite Spuleneinheit (20), die dazu ausgebildet ist, an dem menschlichen oder tierischen Körper positioniert zu werden, um den zweiten Nerv (62) durch Anlegen eines zweiten elektrischen oder elektromagnetischen Feldes zu stimulieren; und
eine Sicherheitsanordnung, die dazu ausgebildet ist, die Einhaltung einer Sicherheitsrichtlinie während der Stimulation des ersten Nervs (62) mittels der ersten Spuleneinheit (10) und des zweiten Nervs (62) mittels der zweiten Spuleneinheit (20) sicherzustellen,
**dadurch gekennzeichnet, dass** die Sicherheitsanordnung eine Messeinheit umfasst, die dazu ausgebildet ist, einen ersten magnetischen Fluss der ersten Spuleneinheit (10) und einen zweiten magnetischen Fluss der zweiten Spuleneinheit (20) zu messen.

2. Die Stimulationsvorrichtung (1) nach Anspruch 1, wobei die Sicherheitsrichtlinie das Aufheben eines elektrischen oder elektromagnetischen Feldes in einem zu dem ersten und dem zweiten Nerv (62) versetzten Bereich umfasst, wenn die erste Spuleneinheit (10) und die zweite Spuleneinheit (20) das erste und das zweite Feld anlegen.

3. Die Stimulationsvorrichtung (1) nach einem der vorangehenden Ansprüche, wobei die erste Spuleneinheit (10) und die zweite Spuleneinheit (20) derart angeordnet sind, dass eine Summe der von der ersten Spuleneinheit (10) und der zweiten Spuleneinheit (20) erzeugten ersten und zweiten Felder bei einem Abstand von mehr als 30 oder 45 cm etwa null beträgt.

4. Die Stimulationsvorrichtung (1) nach einem der vorangehenden Ansprüche, wobei die Sicherheitsanordnung eine Steuereinheit umfasst.

5. Die Stimulationsvorrichtung (1) nach Anspruch 4, wobei die Steuereinheit der Sicherheitsanordnung dazu ausgebildet ist, eine Nichteinhaltung der Sicherheitsrichtlinie zu erkennen und die Aktivierung der ersten Spuleneinheit (10) und der zweiten Spuleneinheit (20) zu verhindern, wenn die Nichteinhaltung erkannt wird, und/oder die erste Spuleneinheit (10) und die zweite Spuleneinheit (20) zu aktivieren, falls die Sicherheitsrichtlinie eingehalten wird.

6. Die Stimulationsvorrichtung (1) nach einem der vorangehenden Ansprüche, die dazu ausgebildet ist, die erste Spuleneinheit (10) und die zweite Spuleneinheit (20) durch Zuführen eines Stimulationsstroms zu der ersten Spuleneinheit (10) und der zweiten Spuleneinheit (20) zu aktivieren.

7. Die Stimulationsvorrichtung (1) nach einem der vorangehenden Ansprüche, wobei die Sicherheitsanordnung eine Sensoreinheit umfasst, die dazu ausgebildet ist, Positionen der ersten Spuleneinheit (10) und der zweiten Spuleneinheit (20) relativ zueinander zu bestimmen, wobei die Sicherheitsrichtlinie vorzugsweise umfasst, dass die relativen Positionen innerhalb eines vordefinierten Bereichs liegen.

8. Die Stimulationsvorrichtung (1) nach einem der vorangehenden Ansprüche,
wobei die Sicherheitsrichtlinie umfasst, dass verhindert wird, dass eine Summe des ersten und des zweiten magnetischen Flusses einen vordefinierten Schwellenwert überschreitet, und/oder
wobei die Stimulationsvorrichtung (1) dazu ausgebildet ist, den ersten und den zweiten magnetischen Fluss durch Messen eines durch die erste Spuleneinheit (10) und die zweite Spuleneinheit (20) fließenden Stroms zu messen, und/oder
wobei die Stimulationsvorrichtung (1) dazu ausgebildet ist, den ersten und den zweiten magnetischen Fluss unter Verwendung eines ersten Teils einer Vielzahl von aufeinanderfolgenden Wellenformen eines Stimulationsstroms zu messen, wobei die Sicherheitsanordnung dazu ausgebildet ist, die Stimulation zu deaktivieren, wenn die Summe des ersten und des magnetischen Flusses den vordefinierten Schwellenwert überschreitet.

9. Die Stimulationsvorrichtung (1) nach einem der vorangehenden Ansprüche, wobei die Sicherheitsanordnung eine Überwachungseinheit umfasst, die dazu ausgebildet ist, einen Fehler der ersten Spuleneinheit (10) und/oder der zweiten Spuleneinheit (20) zu erkennen, wobei die Sicherheitsrichtlinie vorzugsweise umfasst, dass die Überwachungseinheit den Fehler der ersten Spuleneinheit (10) und/oder der zweiten Spuleneinheit (20) nicht erkennt.

10. Die Stimulationsvorrichtung (1) nach einem der vorangehenden Ansprüche, ferner umfassend
einen Verbinder, der dazu ausgebildet ist, sich elektrisch mit der ersten Spuleneinheit (10) und der zweiten Spuleneinheit (20) in Reihe zu verbinden, und/oder
eine Taste, die dazu ausgebildet ist, bei Betätigung die erste Spuleneinheit zu betreiben, um einen Impuls des ersten Feldes zu induzieren, und die zweite Spuleneinheit zu betreiben, um einen Impuls des zweiten Feldes zu induzieren.

11. Die Stimulationsvorrichtung (1) nach einem der vorangehenden Ansprüche, wobei die erste Spuleneinheit (10) und die zweite Spuleneinheit (20) einzeln an dem menschlichen oder tierischen Körper positionierbar sind.

12. Die Stimulationsvorrichtung (1) nach einem der vorangehenden Ansprüche, die derart ausgebildet ist, dass jede von der ersten Spuleneinheit (10) und der zweiten Spuleneinheit (20) biphasische Impulse erzeugt.

13. Die Stimulationsvorrichtung (1) nach einem der vorangehenden Ansprüche,
die dazu ausgebildet ist, zeitveränderliche Ströme in der ersten Spuleneinheit (10) und der zweiten Spuleneinheit (20) zu erzeugen, die zeitlich synchron sind, und/oder
umfassend eine Stimulatoreinheit, die dazu ausgebildet ist, Ströme in der ersten Spuleneinheit (10) und der zweiten Spuleneinheit (20) zu induzieren, und/oder
die derart ausgebildet ist, dass ein in der ersten Spuleneinheit (10) induzierter Strom gleich einem in der zweiten Spuleneinheit (20) induzierten Strom ist, und/oder
die dazu ausgebildet ist, durch einen Strom erzeugte Impulse zu induzieren.

14. Die Stimulationsvorrichtung (1) nach einem der vorangehenden Ansprüche, wobei die Sicherheitsanordnung eine erste Temperaturerfassungsformation und eine zweite Temperaturerfassungsformation umfasst, und wobei die erste Spuleneinheit (10) die erste Temperaturerfassungsformation umfasst und die zweite Spuleneinheit (20) die zweite Temperaturerfassungsformation umfasst,
wobei die erste Temperaturerfassungsformation vorzugsweise einen ersten Temperatursensor und einen zweiten Temperatursensor umfasst, und wobei die zweite Temperaturerfassungsformation einen ersten Temperatursensor und einen zweiten Temperatursensor umfasst, wobei insbesondere die Sicherheitsrichtlinie vorzugsweise umfasst, dass Stromaufnahmen des ersten Temperatursensors der ersten Temperaturerfassungsformation, des zweiten Temperatursensors der ersten Temperaturerfassungsformation, des ersten Temperatursensors der zweiten Temperaturerfassungsformation und des zweiten Temperatursensors der zweiten Temperaturerfassungsformation innerhalb eines vordefinierten Schwellenwertbereichs der Stromaufnahme liegen;
und/oder
wobei die Sicherheitsrichtlinie vorzugsweise umfasst, dass von der ersten Temperaturerfassungsformation und von der zweiten Temperaturerfassungsformation gemessene Temperaturen unterhalb einer vordefinierten Schwellentemperatur liegen.

15. Die Stimulationsvorrichtung (1) nach einem der vorangehenden Ansprüche, wobei die Sicherheitsanordnung ein Zählglied umfasst, das dazu ausgebildet ist, eine Anzahl von durch die erste Spuleneinheit (10) und die zweite Spuleneinheit (20) induzierten Impulsen zu zählen, wobei die Sicherheitsrichtlinie vorzugsweise umfasst, dass die gezählten Anzahlen von Impulsen pro Zeit unterhalb einer vordefinierten Schwellenimpulsanzahl liegen.

## Revendications

1. Un dispositif de stimulation (1) pour stimuler un premier nerf (62) et un deuxième nerf (62) dans un corps humain ou animal, pour activer un tissu cible dans le corps humain ou animal, comprenant :
une première unité de bobine (10) configurée pour être positionnée au niveau du corps humain ou animal, pour stimuler le premier nerf (62) en appliquant un premier champ électrique ou électromagnétique ;
une deuxième unité de bobine (20) configurée pour être positionnée au niveau du corps humain ou animal, pour stimuler le deuxième nerf (62) en appliquant un deuxième champ électrique ou électromagnétique ; et
un agencement de sécurité configuré pour assurer le respect d'une politique de sécurité pendant la stimulation du premier nerf (62) au moyen de la première unité de bobine (10) et du deuxième nerf (62) au moyen de la deuxième unité de bobine (20),
**caractérisé en ce que** l'agencement de sécurité comprend une unité de mesure configurée pour mesurer un premier flux magnétique de la première unité de bobine (10) et un deuxième flux magnétique de la deuxième unité de bobine (20).

2. Le dispositif de stimulation (1) selon la revendication 1, dans lequel la politique de sécurité comprend l'annulation d'un champ électrique ou électromagnétique dans une région décalée par rapport au premier et au deuxième nerf (62) lorsque la première unité de bobine (10) et la deuxième unité de bobine (20) appliquent les premier et deuxième champs.

3. Le dispositif de stimulation (1) selon l'une quelconque des revendications précédentes, dans lequel la première unité de bobine (10) et la deuxième unité de bobine (20) sont agencées de telle sorte qu'une somme des premier et deuxième champs générés par la première unité de bobine (10) et la deuxième unité de bobine (20) soit sensiblement nulle à une distance supérieure à 30 ou 45 cm.

4. Le dispositif de stimulation (1) selon l'une quelconque des revendications précédentes, dans lequel l'agencement de sécurité comprend une unité de commande.

5. Le dispositif de stimulation (1) selon la revendication 4, dans lequel l'unité de commande de l'agencement de sécurité est configurée pour détecter une non-conformité à la politique de sécurité et pour empêcher l'activation de la première unité de bobine (10) et de la deuxième unité de bobine (20) lorsque la non-conformité est détectée, et/ou pour activer la première unité de bobine (10) et la deuxième unité de bobine (20) dans le cas où la politique de sécurité est respectée.

6. Le dispositif de stimulation (1) selon l'une quelconque des revendications précédentes, configuré pour activer la première unité de bobine (10) et la deuxième unité de bobine (20) en fournissant un courant de stimulation à la première unité de bobine (10) et à la deuxième unité de bobine (20).

7. Le dispositif de stimulation (1) selon l'une quelconque des revendications précédentes, dans lequel l'agencement de sécurité comprend une unité de capteur configurée pour déterminer des positions de la première unité de bobine (10) et de la deuxième unité de bobine (20) l'une par rapport à l'autre, dans lequel la politique de sécurité comprend de préférence le fait que les positions relatives se situent dans une plage prédéfinie.

8. Le dispositif de stimulation (1) selon l'une quelconque des revendications précédentes,
dans lequel la politique de sécurité comprend le fait d'empêcher qu'une somme du premier et du deuxième flux magnétique dépasse un seuil prédéfini, et/ou
dans lequel le dispositif de stimulation (1) est configuré pour mesurer les premier et deuxième flux magnétiques en mesurant un courant circulant à travers la première unité de bobine (10) et la deuxième unité de bobine (20), et/ou
dans lequel le dispositif de stimulation (1) est configuré pour mesurer les premier et deuxième flux magnétiques en utilisant une première partie d'une pluralité de formes d'onde consécutives d'un courant de stimulation, dans lequel l'agencement de sécurité est configuré pour désactiver la stimulation lorsque la somme du premier et du flux magnétique dépasse le seuil prédéfini.

9. Le dispositif de stimulation (1) selon l'une quelconque des revendications précédentes, dans lequel l'agencement de sécurité comprend une unité de surveillance configurée pour détecter un défaut de la première unité de bobine (10) et/ou de la deuxième unité de bobine (20), dans lequel la politique de sécurité comprend de préférence le fait que l'unité de surveillance ne détecte pas le défaut de la première unité de bobine (10) et/ou de la deuxième unité de bobine (20).

10. Le dispositif de stimulation (1) selon l'une quelconque des revendications précédentes, comprenant en outre
un connecteur configuré pour se connecter électriquement à la première unité de bobine (10) et à la deuxième unité de bobine (20) en série, et/ou
un bouton configuré pour, lorsqu'il est activé, actionner la première unité de bobine pour induire une impulsion du premier champ et actionner la deuxième unité de bobine pour induire une impulsion du deuxième champ.

11. Le dispositif de stimulation (1) selon l'une quelconque des revendications précédentes, dans lequel la première unité de bobine (10) et la deuxième unité de bobine (20) sont positionnables individuellement au niveau du corps humain ou animal.

12. Le dispositif de stimulation (1) selon l'une quelconque des revendications précédentes, configuré de telle sorte que chacune parmi la première unité de bobine (10) et la deuxième unité de bobine (20) génère des impulsions biphasiques.

13. Le dispositif de stimulation (1) selon l'une quelconque des revendications précédentes,
configuré pour générer des courants variables dans le temps dans la première unité de bobine (10) et la deuxième unité de bobine (20), en synchronisme temporel, et/ou
comprenant une unité stimulatrice configurée pour induire des courants dans la première unité de bobine (10) et la deuxième unité de bobine (20), et/ou
configuré de telle sorte qu'un courant induit dans la première unité de bobine (10) soit identique à un courant induit dans la deuxième unité de bobine (20), et/ou
configuré pour induire des impulsions générées par un courant.

14. Le dispositif de stimulation (1) selon l'une quelconque des revendications précédentes, dans lequel l'agencement de sécurité comprend une première formation de détection de température et une deuxième formation de détection de température, et dans lequel la première unité de bobine (10) comprend la première formation de détection de température et la deuxième unité de bobine (20) comprend la deuxième formation de détection de température,
dans lequel la première formation de détection de température comprend de préférence un premier capteur de température et un deuxième capteur de température, et dans lequel la deuxième formation de détection de température comprend un premier capteur de température et un deuxième capteur de température, en particulier dans lequel la politique de sécurité comprend de préférence le fait que des consommations de courant du premier capteur de température de la première formation de détection de température, du deuxième capteur de température de la première formation de détection de température, du premier capteur de température de la deuxième formation de détection de température, et du deuxième capteur de température de la deuxième formation de détection de température se situent dans une plage de seuil prédéfinie de consommation de courant ;
et/ou
dans lequel la politique de sécurité comprend de préférence le fait que des températures mesurées par la première formation de détection de température et par la deuxième formation de détection de température sont inférieures à une température de seuil prédéfini.

15. Le dispositif de stimulation (1) selon l'une quelconque des revendications précédentes, dans lequel l'agencement de sécurité comprend un élément compteur configuré pour compter un nombre d'impulsions induites par la première unité de bobine (10) et la deuxième unité de bobine (20), dans lequel la politique de sécurité comprend de préférence le fait que les nombres comptés d'impulsions par unité de temps sont inférieurs à un nombre seuil prédéfini d'impulsions.
